# EUROPEAN PATENT APPLICATION

(11) **EP 2 554 042 A1**
(43) Date of publication of application: **06.02.2013**
(21) Application number: 10849006.1
(22) Date of filing: 25.10.2010
(51) Int. Cl.: A01G 7/00, A01G 31/00, A23L 1/30, A23L 1/304, A61K 9/08, A61K 33/26, A61K 47/46, C05D 9/02

(54) **WATER-SOLUBLE IRON SUPPLY AGENT WITH REDUCING POWER AND WITH COFFEE GROUNDS OR TEA DREGS AS RAW MATERIAL**

(30) Priority: 31.03.2010 JP 2010080614
(71) Applicant: Incorporated Administrative Agency National Agriculture and Food Research Organization, Ibaraki 305-8517 (JP)
(72) Inventor: MORIKAWA Claudio Kendi, Tsu-shi Mie 514-2392 (JP); SHINOHARA Makoto, Tsu-shi Mie 514-2392 (JP)
(74) Representative: Gille Hrabal
(86) International application number: PCT/JP2010/068805
(87) International publication number: WO 2011/121833

(57) **Abstract**

Provided are an iron-supplying agent which can supply a water-soluble iron ion stably for a long period of time even under an alkaline condition where an iron ion tends to become insoluble using a naturally derived raw material, and an iron-supplying agent which can supply a water-soluble iron ion using only a raw material which is safe for ingestion in the human body. Also provided is an iron-supplying agent which can supply a water-soluble iron ion (in particular, a divalent iron ion) using trivalent iron as an iron-supplying source. Also provided are a water-soluble iron-supplying agent including a reaction product as an active ingredient, in which the reaction product is obtained by using roasted and ground coffee beans and/or tea leaves as feedsotcks for supplying a metal ion-solubilizing component, and mixing the feedstocks for supplying the metal ion-solubilizing component with an iron-supplying source containing trivalent iron in the presence of water; specifically, a water-soluble iron-supplying agent which can reduce trivalent iron into a divalent iron ion and maintain the divalent iron ion to supply a water-soluble iron ion (divalent iron ion) stably for a long period of time.

## Description

### Technical Field

The present invention relates to a water-soluble iron-supplying agent including, as an active ingredient, a reaction product obtained by using roasted and ground coffee beans (in particular, coffee grounds) or tea leaves (in particular, tea dregs) as feedstocks for supplying a metal ion-solubilizing component, and mixing the feedstocks for supplying the metal ion-solubilizing component with an iron-supplying source containing trivalent iron in the presence of water. Specifically, the present invention relates to a water-soluble iron-supplying agent which can reduce trivalent iron into a divalent iron ion and maintain the divalent iron ion to supply a water-soluble iron ion (divalent iron ion) stably for a long period of time.
Further, the present invention relates to an iron-supplying agent for plant cultivation, which can supply water-soluble iron ion stably for a long period of time in cultivation of a plant (in particular, hydroponic culture or cultivation in alkaline soil). In addition, the present invention relates to an iron-supplying agent which can supply water-soluble iron ion by oral ingestion when used in foods and pharmaceuticals.

### Background Art

Iron is an essential nutrient element for plants, and iron deficiency causes significant deterioration of growth of the plants. However, alkaline soil comprises approximately a third of the land surface of the world in which iron deficiency is common. As a result, most of the land is inappropriate for crop cultivation.
Therefore, as a material for solving this problem, an iron-supplying agent which contains chelated iron (a compound which maintains an iron ion as a complex at multiple ligands) as an active ingredient has been developed.
For example, there is known an iron-supplying agent utilizing, for example, EDTA having a strong iron chelating effect (see Non Patent Literature 1). However, these iron-supplying agents lose their chelating abilities in a short period of time and cannot supply iron stably under high alkaline soil condition. Further, these substances are not natural substances and hence cannot be used in the organic agriculture.
Meanwhile, there is known an iron-supplying agent utilizing a chelated iron of an organic acid derived from a natural product, such as citric acid, malic acid, or lactic acid (see Patent Literature 1). However, the chelated iron by these organic acids which have weak chelating ability easily loses its chelating ability in alkaline soil. Therefore, iron is rapidly insolubilized, and iron cannot be supplied stably.
As mentioned above, under the alkaline condition of alkaline soil, chelating effect disappears and the iron ion released from a chelator becomes insoluble to thereby have a form poorly absorbed by crops.

Therefore, in order to practice organic agriculture in the alkaline soils, it is necessary to develop an iron-supplying agent which is derived from a natural product and can maintain a water-soluble iron ion inexpensively even under an alkaline condition stably for a long period of time. However, such agent has not been found so far, and the practice of organic agriculture in the alkaline soils is very difficult.

In addition, iron is an important nutritional element not only for plants but also for the human body. For example, when iron deficiency is compensated by oral ingestion, symptoms such as hypoferric anemia and allotriophagy can be alleviated in an efficient manner.
However, in food and pharmaceutical fields, an iron-supplying agent which can supply solubilized iron (in particular, a divalent iron ion) stably using only a raw material safe for ingestion in the human body is obtained by an expensive production method, and a technology for producing an excellent iron-supplying agent very inexpensively has been required.

### Citation List

### Patent Literature

[PTL 1] JP-A-2007-195546

### Non Patent Literature

[NPL 1] http://www.nagasechemtex.co.jp/products/nousuisankinzokuen.pdf

### Summary of Invention

### Technical Problem

One object of the present invention is to solve the above-mentioned problems to produce an iron-supplying agent which can supply a water-soluble iron ion stably for a long period of time even under an alkaline condition where an iron ion tends to become insoluble using a naturally derived raw material. Another object of the present invention is to produce an iron-supplying agent which can supply a water-soluble iron ion using only a raw material which is safe for ingestion in the human body.
Another object of the present invention is to produce an iron-supplying agent which can supply a water-soluble iron ion (in particular, a divalent iron ion) using trivalent iron as an iron-supplying source.
Another object of the present invention is to produce the iron-supplying agents very inexpensively.

### Solution to Problem

The inventors of the present invention have made intensive studies in view of such circumstances, and have found that a component in roasted and ground coffee beans or tea leaves can reduce trivalent iron into a divalent iron ion and maintain the divalent iron ion to supply the ion as a water-soluble iron ion stably for a long period of time.

The present invention has been made on the basis of such findings.
That is, a first aspect of the present invention relates to a water-soluble iron-supplying agent including a reaction product as an active ingredient, in which the reaction product is obtained by using roasted and ground coffee beans and/or tea leaves as feedstocks for supplying a metal ion-solubilizing component, and mixing the feedstocks for supplying the metal ion-solubilizing component with an iron-supplying source containing trivalent iron in the presence of water.
Further, a second aspect of the present invention relates to a water-soluble iron-supplying agent according to the first aspect, in which the feedstocks for supplying the metal ion-solubilizing component are coffee grounds.
Further, a third aspect of the present invention relates to a water-soluble iron-supplying agent according to the first aspect, in which the feedstocks for supplying the metal ion-solubilizing component are tea dregs.
Further, a fourth aspect of the present invention relates to a water-soluble iron-supplying agent according to any one of the first to the third aspects, in which the iron-supplying source is a compound which generates a trivalent iron ion.
Further, a fifth aspect of the present invention relates to a water-soluble iron-supplying agent according to any one of the first to the third aspects, in which the iron-supplying source is soil, and the mixing is carried out at 40 to 200°C.
Further, a sixth aspect of the present invention relates to a water-soluble iron-supplying agent according to any one of the first to the fifth aspects, in which the iron-supplying source is mixed so that an iron element is contained in an amount of 0.1 to 10 parts by weight with respect to 100 parts by weight of a dried product of the feedstocks for supplying the metal ion-solubilizing component.
In addition, a seventh aspect of the present invention relates to a water-soluble iron-supplying agent according to any one of the first to the sixth aspects, characterized in that the water-soluble iron-supplying agent is for plant cultivation.
Further, an eighth aspect of the present invention relates to plant cultivation method, characterized by including adding the water-soluble iron-supplying agent according to the seventh aspect to alkaline soil.
Moreover, a ninth aspect of the present invention relates to a water-soluble iron-supplying agent according to any one of the first to the sixth aspects, characterized in that the water-soluble iron-supplying agent is for oral ingestion.

### Advantageous Effects of Invention

According to the present invention, it is possible to produce a water-soluble iron-supplying agent which can maintain a water-soluble iron ion (specifically, a divalent iron ion) stably for a long period of time even under an alkaline condition, where an iron ion tends to become insoluble, using trivalent iron as an iron-supplying source.
Further, according to the present invention, it is possible to produce a water-soluble iron-supplying agent inexpensively using trivalent iron which is in abundant supply in nature (in particular, soil-derived trivalent iron) as an iron-supplying source.
Further, according to the present invention, it is possible to produce the water-soluble iron-supplying agent more inexpensively using coffee grounds or tea dregs as the feedstocks for supplying the metal ion-solubilizing component.
It should be noted that coffee and tea drinks are the mostly consumed beverages in the world, and the coffee grounds and tea dregs which are waste materials thereof are daily produced in large amounts throughout the world. However, the waste materials are used only in composts, deodorants, and the like, and a novel effective use method for these materials has been searched. Therefore, the present invention is expected to contribute to the effective use of these wastes, by creating a novel application method for the food waste materials.

As a result, according to the present invention, it is possible to provide an iron-supplying agent for plant cultivation and alleviate iron deficiency in alkaline soil or hydroponics. Further, the agent can be produced using only a naturally derived raw material and is hence very safe for an environment, and the agent is expected to be used as a technology for enabling organic agriculture in alkaline soil.

In addition, according to the present invention, it is possible to provide an iron-supplying agent for oral ingestion, which is very safe for the human body when used in foods and pharmaceuticals, and the agent is expected to contribute to the alleviation of various symptoms caused by iron deficiency.

### Brief Description of Drawings

[FIG. 1] A photographic image of a divalent iron ion solubilized through reduction from trivalent iron in soil by a component derived from coffee grounds and detected by using dipyridyl in Example 1.
[FIG. 2] Aphotographic image showing an effect of a reaction product of coffee grounds and iron on plant cultivation in alkaline soil in Example 2.

### Description of Embodiments

The present invention relates to a water-soluble iron-supplying agent including, as an active ingredient, a reaction product obtained by mixing a specific feedstock for supplying a metal ion-solubilizing component with an iron-supplying source containing trivalent iron in the presence of water.

### (Feedstock for supplying metal ion-solubilizing component)

Roasted and ground coffee beans or tea leaves may be used as the feedsotcks for supplying the metal ion-solubilizing component in the present invention. Further, a mixture thereof may be used.
In addition, an extracted component obtained by immersing the roasted and ground coffee beans or tea leaves (in particular, coffee grounds or tea dregs) in water (so-called soluble component of coffee or tea or soluble component of coffee grounds or tea dregs) or only insoluble components (so-called coffee grounds or tea dregs) may be used as the feedstocks for supplying the metal ion-solubilizing component. It should be noted that the components may be dried into powder before use.
The component is a composition containing various molecular species of phenols and polyphenols, and having both an effect of reducing trivalent iron into a divalent iron ion and an effect of maintaining the divalent iron ion for a long period of time.
It should be noted that trivalent iron tends to become insoluble depending on the kind of the iron-supplying source or a condition (in particular, an alkaline condition), but a divalent iron ion reduced from trivalent iron is in a state of being easily dissolved in water and easily absorbed in animals and plants.

Any of coffee beans roasted and ground by a usual method may be used as the 'roasted and ground coffee beans' used in the present invention. So-called ground coffee beans are also included. Further, coffee beans which are roasted after grinding may be used.
In this case, any seeds of *Coffea* such as *Coffea Arabica* (arabic), C. *canephora* (Robusta), or *C*. *liberica* (Liberian coffee) may be used as the coffee beans. It should be noted that raw coffee beans, and coffee beans dried and preserved as usual may be used.
Further, from the viewpoint of the cost of raw materials, off-specification coffee beans are industrially preferably used. In particular, 'coffee grounds' wasted in large amounts after extraction of coffee are most preferably used.

It should be noted that roasting may be carried out by any of usual methods. Examples thereof include open fire roasting, hot air roasting, far-infrared roasting, microwave roasting, heated steam roasting, and low temperature roasting.
Further, grinding has only to be carried out so that usual ground coffee beans are obtained by using a coffee mill, a grinder, a stone mill, or the like, and the level of grinding widely ranges from coarsely grinding to grinding into powder. It should be noted that the grinding includes a treatment such as fracture, pulverization, or powderization. From the viewpoint of the reaction efficiency with iron, powderized coffee beans having small particle sizes are preferred.

Any picked stems and leaves of Camellia *sinensis* which is one of the tea plants may be used as the 'tea leaves' used in the present invention. Further, a method of picking the leaves may be any one, but from the viewpoint of the cost, mechanical picking is particularly preferred.
It should be noted that, in the picked tea leaves, cell contents are mixed together to cause oxidative fermentation, but tea leaves at any stage of fermentation may be used here. For example, there may be used: green tea obtained by suppressing oxidative fermentation by heating (such as green tea of middle grade, coarse green tea, twig tea, or roasted green tea); blue tea obtained by fermenting the leaves to some degree (such as oolong tea); black tea obtained by completely fermenting the leaves; and dark tea (such as pu'er tea) obtained by oxidative fermentation and fermentation with a aspergilli. Preferred examples thereof include green tea, black tea, and oolong tea.
It should be noted that, from the viewpoint of the cost of raw materials, off-specification tea leaves are industrially preferably used. In particular, 'tea dregs' wasted in large amounts after extraction of tea are most preferably used.

### (Iron-supplying source)

In the present invention, any iron-supplying source including trivalent iron may be used.
Examples thereof include: a water-soluble iron compound such as iron (III) chloride or iron (III) sulfate; an insoluble iron compound such as iron (III) oxide, iron (III) nitrate, or iron (III) hydroxide; and a natural product such as soil (in particular, iron-rich soil such as Akadama soil, Kanuma soil, or allophanic loam, or soil containing amorphous mineral (in particular, geothite)), heme iron, or seashell. Further, examples thereof include a product obtained by dissolving iron ore (natural iron ore such as pyrite, marcasite, siderite, magnetite, or goethite), an iron material (metallic iron), or red soil (iron (III) oxide-rich soil such as laterite) with an acid. Further, rust may be used as a source. In addition, an aqueous solution obtained by dissolving a water-soluble iron compound and containing a trivalent iron ion may be used.
Of those, in the case where the agent is used in the fields of food, medicine, and the like, an inexpensive iron compound (a trivalent iron compound such as iron (III) chloride or iron (III) sulfate) is preferably used from the viewpoints of the cost of raw materials and quality assurance.
Moreover, in the case where the agent is used in organic agriculture, a natural product of soil (in particular, Akadama soil, Kanuma soil, or allophanic loam) is preferably used as the iron-supplying source from the viewpoints of the necessity to limit a raw material only to a natural product, cost of raw materials, and stable supply.

### (Mixing treatment)

In the present invention, when the feedstock for supplying the metal ion-solubilizing component (or the component derived from the raw material) is mixed with the iron-supplying source (or a trivalent iron ion) in the presence of water, a reaction product capable of supplying a water-soluble iron ion (divalent iron ion) stably for a long period of time can be obtained.

In this case, the expression "in the presence of water" refers to a condition where a component having a reducing power contained in the feedstock for the metal ion-solubilizing component can react with iron using water as a medium.
For example, the amount of water used may be one enough for wetting the raw materials by a mixing procedure, and is desirably, for example, 0.5 part by mass or more, preferably 2 parts by mass or more with respect to a total of 1 part by mass of the raw materials. It should be noted that the upper limit may be an amount which enables the mixing procedure, and is, for example, 100 parts by mass or less, preferably 10 parts by mass or less.
It should be noted that water may be any usual water, and examples thereof include well water, river or lake water, seawater, tap water, deionized water, and distilled water. Further, water containing a pH buffer, a salt (such as NaCl or KCl), an alcohol (suchasethanol), a saccharide, an acid, or an alkali may be used as long as the condition allows the reaction to occur.

With regard to a mixing ratio of raw materials, the iron-supplying source may be mixed in an amount of 0.1 part by weight or more, preferably 4 parts by weight or more in terms of the weight of an iron element with respect to 100 parts by weight of the dried product of the feedstocks for supplying the metal ion-solubilizing component. If the ratio of iron is too low, iron adsorbs to the feedstocks for supplying the metal ion-solubilizing component, which is not preferred.
In addition, the upper limit is 10 parts by weight or less, preferably 5 parts by weight or less. If the ratio of iron is too high, unreduced trivalent iron remains, which is not preferred.

In this case, with regard to a mixing procedure, mixing may be carried out simply by stirring, but may be carried out with a mixer, a large-scale stirring vessel, a Vortex mixer, a shaker, or the like.
In this case, the temperature of water may be one where water is in a liquid state (for example, 1 to 100°C), but mixing can be carried out at about room temperature (for example, 10 to 40°C) without particular heating.
It should be noted that, in the case where a specific natural product (specifically soil) is used as the iron-supplying source, or in the case where the iron-supplying source is mainly composed of an insoluble iron compound, it is necessary to adjust the temperature of water used in mixing to 40°C or more, preferably 50 °C or more to facilitate the reaction between iron and the metal ion-solubilizing component. It should be noted that the upper limit is 200°C (in the case of heating under increased pressure), but from the viewpoint of production cost, the temperature is preferably 100°C, the boiling point of water in usual heating, or less, more preferably 70°C or less. It should be noted that, in order to suppress thermal degradation of the metal ion-solubilizing component under a reaction condition of 100°C or more, it is more effective to carry out mixing in a sealed container.

With regard to a mixing time, mixing has only to be carried out for about 10 seconds or more until the metal ion-solubilizing component is brought sufficiently into contact with iron, but in order to improve uniformity, a mixing treatment is carried out desirably for 3 minutes or more.
With regard to the upper limit, in order to prevent putrescence due to propagation of microorganisms, mixing is desirably terminated within 240 hours. However, in the case where a sterilization treatment is carried out, the upper limit is not particularly specified.

### (Water-soluble iron-supplying agent)

The reaction product obtained by the above-mentioned steps (roasted and ground coffee or tea leaves after the mixing treatment) has an ability to maintain and supply a divalent iron ion (water-soluble iron ion), which is obtained by reducing trivalent iron derived from the iron-supplying source, stably for a long period of time. In addition, the ability to supply a water-soluble iron ion is exerted stably even under an alkaline condition.
Therefore, the reaction product has an excellent ability as an active ingredient of the 'water-soluble iron-supplying agent.'

The reaction product obtained by the steps may be used as the active ingredient of the water-soluble iron-supplying agent without additional treatments (in a liquid or water-containing state) or after drying (such as natural drying or roasting). Further, a supernatant or suspension obtained by dissolving the dried product in water may be used as the active ingredient.
It should be noted that a liquid obtained by collecting only the supernatant from the resultant reaction product (or a dried product thereof) may be used as the active ingredient.

### (Application to plant cultivation)

The water-soluble iron-supplying agent of the present invention can be used as a water-soluble iron ion-supplying agent for plant cultivation. Further, the agent can be produced from a naturally derived raw material alone, and is hence very safe for an environment. It should be noted that the water-soluble iron ion which can be supplied by the water-soluble iron-supplying agent is a divalent iron ion and is hence suitable for absorption in plants.

The water-soluble iron-supplying agent can be used for any usual plant cultivation in agriculture and gardening. In particular, the agent can be effectively used in hydroponic culture (hydroponics) or cultivation in alkaline soil where iron deficiency tends to occur. It should be noted that, in view of usefulness intended for improvement of crops or the like, the agent is most effectively used in the alkaline soil.
Here, the alkaline soil refers to soil under an alkaline condition of about pH 7 to 10, and examples thereof include calcareous alkaline soil. However, in the present invention, the agent may be used in strongly alkaline soil of pH 9 or more. It should be noted that, under such conditions, conventional general iron-supplying agents such as chelators (such as EDTA iron and iron citrate) cannot be used at all.

Examples of the formof the agent include solid and liquid forms. Examples thereof include powder, granule, sheet, board, cube, sponge, concentrate, and liquid ampule forms. Examples thereof further include a powdery form, a form of a solid mixed with an excipient or the like, a form of a capsule filled with the agent, and a gel.
With regard to the form of the agent used, in the case where the agent is a liquid, the agent is used with or without dilution, while in the case where the agent is a solid, the agent is dissolved in water or the like before use.
Specifically, the water-soluble iron-supplying agent of the present invention may be used, for example, by: burying the agent in soil for cultivation; adding the agent to soil for cultivation; spraying the agent in a liquid state to soil for cultivation; adding the agent to soil for cultivation to prepare mixed soil; adding the agent to a nutrient solution for hydroponic culture; performing foliar spray to a plant; or injecting the agent into a plant.
The amount of the water-soluble iron-supplying agent used is, for example, 0.01 to 50 g/L (content per soil volume) in the case where the reaction product used as an active ingredient is added to soil, or 0.01 to 50 g/L (concentration per nutrient solution) in the case where the reaction product used as an active ingredient is added to a nutrient solution for hydroponic culture.

### (Application to pharmaceutical and food)

In addition, the water-soluble iron-supplying agent of the present invention can be used as a water-soluble iron ion-supplying agent for oral ingestion when used in foods and pharmaceuticals. Further, the agent can be produced using a naturally derived raw material and is hence very safe for the human body. It should be noted that the water-soluble iron ion which can be supplied by the water-soluble iron-supplying agent is a divalent iron ion and is hence suitable for absorption in the human body.

The water-soluble iron-supplying agent can be used as a pharmaceutical and can alleviate various symptoms caused by a iron deficiency. For example, the agent can alleviate symptoms such as hypoferric anemia and allotriophagy effectively.
It should be noted that the water-soluble iron-supplying agent may be added to a food or beverage and used as a functional food or beverage.

Examples of the form of the agent include solid and liquid forms. Examples thereof include powder, granule, suspension, extract, concentrate, and liquid ampule forms. Examples thereof further include a powdery form, a form of a solid mixed with an excipient or the like, a form of a capsule filled with the agent, and a form of a gel mixed with the agent.
In addition, a functional food or beverage which may contain the agent may be obtained by mixing the agent with various food raw materials, for example, by adding the agent to tablets (chewable tablets (such as supplements)), biscuits, snack foods, candies, jellies, ice creams, toppings (furikake), soft drinks, and drinks.

With regard to an effective ingestion dose, the water-soluble iron ion can be supplied sufficiently by orally ingesting the above-mentioned reaction product used as an active ingredient in an amount of 10 mg or more, preferably 200 mg or more per adult with a body weight of 60 kg per day. Therefore, the water-soluble iron-supplying agent can be ingested by a form and an ingestion method (frequency, amount) sufficient for the amount needed.
However, it is desirable to appropriately select the form and ingestion method depending on, for example, the age and body weight of a subject, symptom, ingestion schedule, or form of a preparation.

### Examples

Hereinafter, the present invention is described by way of examples, but the scope of the present invention is not limited by these examples.

### (Example 1) Examination of ability to reduce iron of component in coffee grounds and ability to solubilize iron thereof

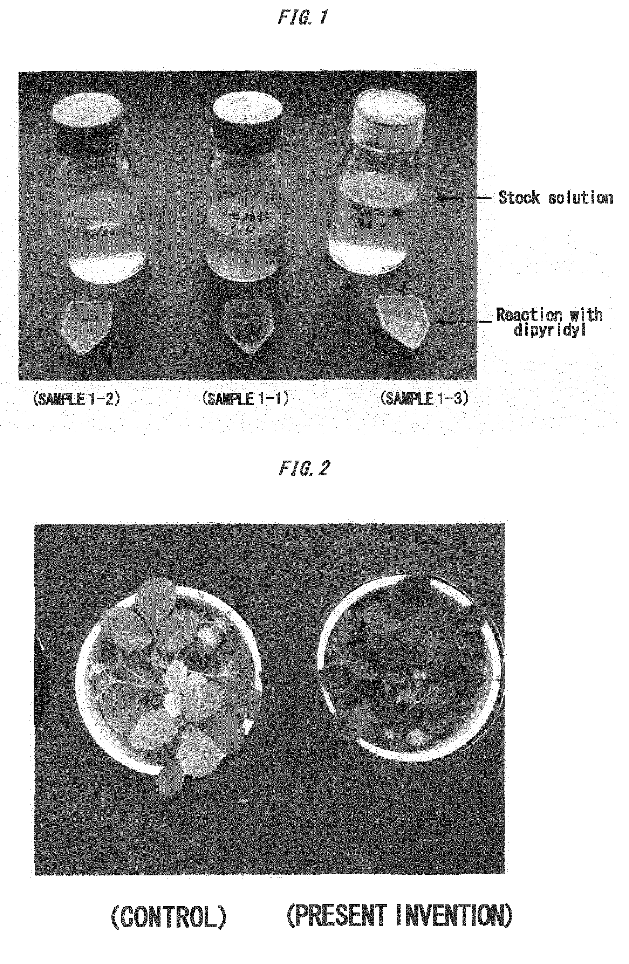

An experiment on solubilization of trivalent iron contained in Akadama soil through reduction into a divalent iron ion was carried out using coffee grounds.
1, 200 g of Akadama soil were mixed with 800 g of coffee grounds, and water was added and mixed thereto in twice the total weight of the Akadama soil and coffee grounds. The resultant was allowed to stand still at 60°C overnight to perform a reaction. After the reaction, the resultant was dried by air. Then, 2 g of the resultant product were added to and suspended in 1 L of distilled water (Sample 1-1: coffee grounds/Akadama soil suspension). It should be noted that, as a control, 1.2 g of Akadama soil were suspended alone in 1 L of water to prepare a solution (Sample 1-2: Akadama soil suspension). The solutions were allowed to stand still at room temperature for 44 days.
Further, a solution was prepared as a comparative sample in the same manner as above except that the same weight of citric acid was used instead of the coffee grounds (Sample 1-3: citric acid/Akadama soil suspension). It should be noted that the comparative sample was not allowed to stand still at room temperature for a long period of time.
0.1 ml of 0.2% dipyridyl (dipyridyl 2 g, acetic acid 100 g/L) was added to 3 ml of each of the resultant three solutions, and color development was observed to detect a divalent iron ion. FIG. 1 shows the results. It should be noted that dipyridyl is a substance which turns red when reacted with a divalent iron ion and is used for detection of the divalent iron ion. The substance does not react with trivalent iron (or a trivalent iron ion) and remains colorless.

As a result, a divalent iron ion was detected from Sample 1-1 (coffee grounds/Akadama soil suspension), and the result suggested that the component in the coffee grounds reduced trivalent iron in the Akadama soil into the divalent iron ion and was able to maintain the ion in a water-soluble state stably for a long period of time (44 days).
On the other hand, a divalent iron ion was not detected from Sample 1-3 (citric acid/Akadama soil suspension), and the result suggested that citric acid (having an effect of chelating a divalent iron ion) could not reduce trivalent iron in the Akadama soil into a divalent iron ion to make the iron water-soluble (even immediately after the same production method). In addition, a divalent iron ion was not detected from Sample 1-2 (Akadama soil suspension) as a control.
This suggests that citric acid, conventional chelator, is not applicable for using Akadama soil, but use of coffee grounds can solubilize trivalent ion in Akadama soil, and that the divalent iron ion, which is easily absorbed by plants, can be supplied stably.
It should be noted that it has been conventionally known that chlorogenic acid, tannic acid, and caffeic acid in coffee each have an iron-chelating effect, but the ability of each of the compounds to reduce trivalent iron into divalent iron has not been known.
This is the first to show that the components derived from the coffee grounds each have an effect of reducing trivalent iron into a divalent iron ion. It should be noted that the components each having the reduction effect are considered to be any of phenols or polyphenols.

### (Example 2) Effect of supplying water-soluble iron ion in alkaline soil

First, coffee grounds were allowed to react with Akadama soil in the presence of water in the same manner as in Example 1 and the resultant was dried by air, to thereby obtain a dried product (reaction product of coffee grounds and iron).
Then, the dried product was added to a pot filled with alkaline soil (pH 9.2) at 1 g/kg, and strawberry was cultivated. Meanwhile, strawberry was cultivated in another pot filled with alkaline soil not containing the dried product as a control. FIG. 2 shows the results.

As a result, strong iron deficiency was observed in the pot where the strawberry was cultivated in the alkaline soil alone (FIG. 2, left: control), while the strawberry grew healthily in the pot containing the reaction product of coffee grounds and iron (FIG. 2, right: the present invention).
This shows that an iron ion which can be easily absorbed in plants can be supplied stably by using the reaction product of coffee grounds and iron even in the alkaline soil where iron tends to become insoluble.

### Industrial Applicability

The present invention is expected to be applied in agricultural fields (alleviation of iron deficiency in alkaline soil or hydroponics) as an iron-supplying agent for plant cultivation which is very safe to an environment. The present invention is expected to contribute particularly to increasing food production around the world by using the present invention for soil improvement of alkaline soil.
In addition, the present invention is expected to be applied to food and pharmaceutical fields as an iron-supplying agent for oral ingestion, which is very safe for the human body. The present invention is expected to contribute particularly to the alleviation of various symptoms caused by iron deficiency.
Further, it is expected that novel applications of food wastes such as coffee grounds and tea dregs are created to contribute to an effective use.

## Claims

1. A water-soluble iron-supplying agent, comprising a reaction product as an active ingredient, wherein the reaction product is obtained by using roasted and ground coffee beans and/or tea leaves as feedstocks for supplying a metal ion-solubilizing component, and mixing the feedstocks for supplying the metal ion-solubilizing component with an iron-supplying source containing trivalent iron in the presence of water.

2. A water-soluble iron-supplying agent according to claim 1, wherein the feedsotcks for supplying the metal ion-solubilizing component are coffee grounds.

3. A water-soluble iron-supplying agent according to claim 1, wherein the feedstocks for supplying the metal ion-solubilizing component are tea dregs.

4. A water-soluble iron-supplying agent according to any one of claims 1 to 3, wherein the iron-supplying source is a compound which generates a trivalent iron ion.

5. A water-soluble iron-supplying agent according to any one of claims 1 to 3, wherein the iron-supplying source is soil, and the mixing is carried out at 40 to 200°C.

6. A water-soluble iron-supplying agent according to any one of claims 1 to 5, wherein the iron-supplying source is mixed so that an iron element is contained in an amount of 0.1 to 10 parts by weight with respect to 100 parts by weight of a dried product of the feedstocks for supplying the metal ion-solubilizing component.

7. A water-soluble iron-supplying agent according to any one of claims 1 to 6, wherein the water-soluble iron-supplying agent is for plant cultivation.

8. A plant cultivation method, comprising adding the water-soluble iron-supplying agent according to claim 7 to alkaline soil.

9. A water-soluble iron-supplying agent according to any one of claims 1 to 6, wherein the water-soluble iron-supplying agent is for oral ingestion.
